(19)

Europäisches
Patentamt
European
Patent Office
Office européen
des brevets

(11) **EP 1 979 029 B1**

(12) **EUROPEAN PATENT SPECIFICATION**

(45) Date of publication and mention
of the grant of the patent:
**09.02.2011 Bulletin 2011/06**

(51) Int Cl.:
*A61M 13/00* (2006.01)    *A61M 5/00* (2006.01)

(21) Application number: **05850980.3**

(22) Date of filing: **21.12.2005**

(86) International application number:
**PCT/IT2005/000752**

(87) International publication number:
**WO 2007/072514 (28.06.2007 Gazette 2007/26)**

(54) **APPARATUS AND METHOD FOR SUBCUTANEOUS GAS DELIVERY FOR THERAPEUTIC PURPOSE**

GERÄT UND VERFAHREN ZUR SUBKUTANEN GASABGABE FÜR THERAPEUTISCHE ZWECKE

APPAREIL ET PROCEDE POUR L'ADMINISTRATION SOUS-CUTANEE DE GAZ A DES FINS THERAPEUTIQUES

(84) Designated Contracting States:
**AT BE BG CH CY CZ DE DK EE ES FI FR GB GR
HU IE IS IT LI LT LU LV MC NL PL PT RO SE SI
SK TR**

(43) Date of publication of application:
**15.10.2008 Bulletin 2008/42**

(73) Proprietor: **Carbossiterapia Italiana S.R.L.
20133 Milano (IT)**

(72) Inventor: **PARMIGIANI, Roberto, Maria
I-20013 Magenta (IT)**

(74) Representative: **Petruzziello, Aldo et al
Racheli S.R.L.
Viale San Michele del Carso, 4
20144 Milano (IT)**

(56) References cited:
**DE-A1- 4 319 612      US-A1- 2001 006 812
US-A1- 2005 113 797      US-B1- 6 632 194**

**Description**

The present invention refers to an apparatus for subcutaneous delivery of gas for therapeutic purposes. In particular carbon dioxide ($CO_2$ can be used as the gas.

This type of therapy is typically used in the treatment of peripheral artery disease, hypertension, chronic arthritic and rheumatic conditions and acute inflammatory conditions such as epicondylitis, periarthritis and the like.

More recently, such treatments have also been employed in aesthetic medicine, for the treatment of edematofibrosclerotic panniculitis (EFP) or cellulite.

The apparatus for subcutaneous delivery of gas of the prior art present some drawbacks.

In fact the gas from a compressed gas cylinder is supplied to the apparatus in which it undergoes rapid expansion with a decrease in pressure and consequently a decrease in temperature. As a result, gas at a very low temperature (about -100°C) emerges from the needle and is injected beneath the patient's skin. This leads to problems for the patient who is unlikely to tolerate injection of such a cold gas.

Heating of a gas with electrical resistances passed through by an electric current is known to the art. However, this system involves some drawbacks due to the fact that the heated gas can be a vehicle for germs, bacteria and other impurities which can be propagated therewith within the patient's skin.

US 2005/0113797 discloses an apparatus for subcutaneous delivery of therapeutic gas, comprising a tank, a deliver line and a heating/sterilization system according to the preamble of claim 1.

The object of the present invention is to overcome the drawbacks of the prior art by providing an apparatus for subcutaneous delivery of gas for therapeutic purposes that are practical for the user and at the same time able to allow efficient, comfortable, safe treatment for the patient.

This object is achieved in accordance with the invention with the characteristics listed in appended independent claim 1.

Advantageous embodiments of the invention are apparent from the dependent claims.

The apparatus for subcutaneous delivery of gas for therapeutic purposes, in particular of carbon dioxide ($CO_2$) according to the invention comprises a tank for delivery of the gas connected, by means of a delivery line, to a needle designed to be inserted into the patient's subcutaneous tissue.

The peculiar characteristic of the invention is represented by the fact that said apparatus further comprises a heating/sterilization system disposed on the delivery line, upstream of the injection needle, to sterilize the gas and heat it to an optimal temperature to enable injection beneath the patient's skin.

The apparatus according to the invention has two considerable advantages with respect to the prior art. In fact, said apparatus is able to administer gas at ambient temperature, making the treatment much more comfortable for the patient. Furthermore, the gas is sterilized, preventing it from being able to form a vehicle for germs, bacteria or other pathogenic agents harmful to humans.

Further characteristics of the invention will be made clearer by the detailed description that follows, referring to a purely exemplifying and therefore non-limiting embodiment thereof, illustrated in the appended drawings, in which:

Figure 1 is a block diagram diagrammatically illustrating the apparatus for subcutaneous delivery of gas for therapeutic purposes according to the invention;

Figure 2 is a block diagram illustrating in greater detail the gas heating and sterilizing system of the apparatus according to the invention;

Figure 3 is a diagrammatic view in axial section illustrating the main heater/sterilizer of the heating and sterilizing system according to the invention; and

Figure 4 is a graph showing the temperature values of a gas, according to its flow, at a power of 1W.

The apparatus for subcutaneous delivery of gas for therapeutic purposes according to the invention, denoted as a whole by reference numeral 100, is described with the aid of the figures.

The apparatus 100 comprises a tank 10 containing compressed gas which must be delivered for the treatment. The tank 10 is connected to a delivery line 20. An inlet filter

F1, which acts a safety filter to filter any impurities contained in the tank 10, is disposed in the delivery line 20, at the outlet of the tank 10.

In the delivery line 20, downstream of the inlet filter F1, is disposed an inlet valve V1, downstream of which is disposed a first flow restrictor R1 with a one-way valve U1 in parallel.

Downstream of the flow restrictor R1 is disposed a balance tank 11, downstream of which is disposed an outlet valve V2 and a second flow restrictor R2.

Downstream of the second flow restrictor R2 is disposed a heating and sterilizing system 30 to heat and sterilize the gas, downstream of which are disposed an outlet filter F2 and an injection needle 4 to be inserted in the patient's subcutaneous tissue during delivery of the gas.

The apparatus 100 further comprises a relief valve V3 disposed in a branch 21 of the delivery line 20, between the inlet valve V1 and the first flow restrictor R1. Opening of the relief valve V3 allows the pressure of the gas leaving the balance tank 11 to be lowered. In fact, when the relief valve V3 is open, the gas, following the course of the arrow B, passes through the one-way valve U1 and flows to the outside through the relief valve V3. A silencer 5 is disposed on the outlet of the relief valve V3.

An outlet sensor S1 is disposed in a branch 22 connected in parallel to the outlet valve V2, to the second flow restrictor R2 and to the heating device 30. The outlet sensor S1 is a differential pressure sensor able to measure the pressure difference ($P_C$ - $P_B$), between a pressure $P_C$ at the outlet of the balance tank 11 and a pressure $P_B$ at the inlet of the outlet filter F2. The pressure $P_B$ indicates the load represented by the patient's subcutaneous tissue for the time for which the needle 4 is inserted.

On the other hand an inlet pressure sensor S2 is disposed at the outlet of the tank 10, between the inlet filter F1 and the inlet valve V1.

The apparatus 100 further comprises a user interface comprising a keyboard 1 to enter data, a screen 2 to display data and graphics and a pedal switch 3 to enable operation of the machine.

Lastly, the apparatus 100 comprises a control unit UC connected to all the electrical members: the pressure sensors S1 and S2, the valves V1, V2, V3, the keyboard 1, the screen 2, the enabling switch 3 and the heating and sterilizing system 30.

The control unit UC receives input signals from the outlet S1 and inlet S2 pressure sensors, from the keyboard 1 and from the enabling switch 3, and controls opening and closing of the valves V1, V2, V3 accordingly, so as to adjust the outlet pressure $P_C$ of the balance tank 11 and maintain the gas flow delivered to the apparatus 100 at a constant value entered by the user, also following changes in the load represented by the cutaneous tissue into which the needle 4 is inserted and thus following changes in the pressure $P_B$.

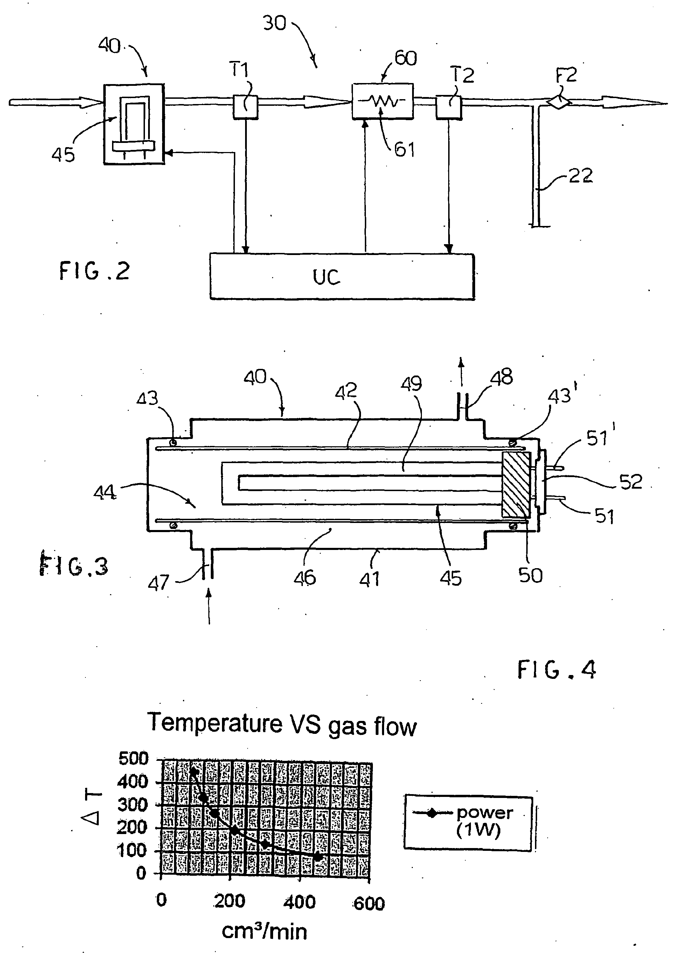

The heating and sterilizing system 30 is described in greater detail hereunder with reference to Figures 2, 3 and 4.

As shown in Figure 2, the heating and sterilizing system 30 comprises a main heater/sterilizer 40, and a temperature regulating system comprising a first temperature sensor T1 disposed at the outlet of the main heater/sterilizer 40, a post-heater 60 and a second temperature sensor T2 disposed at the outlet of the post-heater 60.

As shown in Figure 3, the main heater/sterilizer 40 comprises a substantially cylindrical container 41 inside which is mounted a transparent cylindrical shield 42, by means of gaskets 43, 43' disposed in the smaller diameter ends of the container 41.

In this manner inside the transparent shield 42 a cylindrical gas-tight seat 44 is formed, able to house axially an ultraviolet (UV) lamp 45. On the other hand, between the transparent shield 42 and the container 41 a toroidal space is formed which constitutes a chamber 46 within which the gas of the apparatus 100 can flow.

In the container 41 there are an inlet duct 47 and an outlet duct 48 disposed in a distal position with respect to the inlet duct 47 communicating with the toroidal chamber 46, so that the gas passes through the whole toroidal chamber 46.

The UV lamp 45 has an oblong bulb 49 which extends for a slightly smaller length than that of the transparent shield 42. The bulb 49 is connected to a fitting 50 mounted at one end of the transparent shield 42. Integrated in the fitting 50 is the power supply for the lamp 45 which is connected to two electrical cables 51, 51' which are brought out of the container 41, passing through a flange 52 closing the rear end of the container 41, to be connected to the electrical power supply of the machine 100.

By way of example, the container 41 has an outside diameter of about 6 cm and a length of about 12 cm, whereas the transparent shield 42 has a diameter of about 4 cm. In this manner the toroidal chamber 46 into which the gas flows has a volume of about 170 cm$^3$ and ensures a gas flow of about 150 cm$^3$/min.

The UV lamp 45 is preferably of the low pressure mercury type and emits radiation in the ultraviolet range between 250ηm and 280ηm, with electrical power absorption of 5 Watt. These UV lamps are known as germicidal lamps since they emit radiation capable of eliminating germs, bacteria and other microorganisms or pathogenic agents. In this manner the radiation of the lamp strikes the entire gas flow passing through the chamber 46, allowing adequate sterilization of the gas. However, such a germicidal UV lamp is not capable of heating the flow of gas.

Carbon dioxide, which has an absorption spectrum with peaks concentrated in the infrared region and a main peak at 15$\mu$m, is generally used as the gas in the apparatus 100. Therefore, to be able to heat the gas, the chamber 46 is made like a blackbody cavity in order to convert the high-frequency electromagnetic waves emitted by the UV lamp 45 from the ultraviolet range (between 250ηm and 280ηm) to the infrared range (between 5$\mu$m and 20$\mu$m). In particular, if the wall of the chamber 46 has a temperature of about 30 °C, an emission peak in the infrared region around 10$\mu$m is encountered.

In order to obtain this result, the container 41 which defines the outside wall of the chamber 46, can be made of aluminium, in which the outer surface thereof is left in the natural state of reflective metal, whereas the inner surface is treated by means of a process known by the name of deep oxidation. In this manner a thermos is produced which retains inside it the electromagnetic radiation converted to the infrared range. Thus inside the chamber 46, the energy of the infrared radiation is absorbed by the cold carbon dioxide (at about -100°C) which is heated with a thermal jump of about 120°C, reaching a temperature of about 20°C.

The conversion of UV rays emitted by the lamp 45 into infrared rays takes place inside the chamber 46 on the surface of the inside wall. This phenomenon is explained by the fact that whatever the temperature of a body, it emits a spectrum of electromagnetic waves. The maximum peak (in wavelength) in which emission occurs at a certain temperature of the body is related to Wien's law:

$$\lambda(m) = 0.0029/\ T(Kelvin) \qquad\qquad (1)$$

in which

$\lambda$ is the wavelength expressed in metres, and

T is the temperature in degrees Kelvin (T(Kelvin) = T (°C) + 273)

If the UV rays that strike the inside walls of the chamber 46 heat it, for example to 30°C, on the basis of the formula (1), an infrared field is created inside the chamber with a peak at $10\mu$m, with significant components of the spectrum also at $15\mu$m where carbon dioxide has an absorption peak.

In any case an external heating system which maintains the walls of the chamber 46 at a temperature of 30°C or greater can be provided.

When a gas is subjected to a heat source, the power dissipated is expressed by the following formula:

$$Power(W) = Flow(Moles/sec) * Cp(J/°K\ Mole) * \Delta T(°K) \qquad\qquad (2)$$

In which:

P(W) is the power expressed in Watts

Flow(moles/sec) is the flow of gas expressed in moles per second. In standard conditions 1 mole of carbon dioxide occupies a volume of about 22 litres. Thus, for a flow of 150 cm$^3$/min (in standard conditions), Flow(moles/ sec) = 0.0001

$C_p$ is the specific heat at a constant pressure. For carbon dioxide Cp = 37(J/°K Mole)

Using these data in the formula (2), a theoretical heating of 250°C with 1 W of power and a flow of 150 cm$^3$/min is achieved (as shown in the graph of Figure 4 which shows the theoretical heating values).

In the practical case of the container 41, a system efficiency of about 10% is hypothesized.

Thus, using a light source 45 of 5 W with a gas flow of about 150 cm$^3$/min, a heating of about 120°C is achieved. Thus the cold gas enters the chamber 46 of the first heater at a temperature in the range of -110°C to -100°C; as a result it leaves the chamber 46 at a temperature in the range of 10°C to 20°C.

The staying time of the gas inside the chamber 46 is established solely by the geometry of the chamber and by the flow of the gas. If the power of the lamp 45 remains constant, heating will be greater at low flow rates. In fact, if the temperature of the gas is to be maintained constant, it will be necessary to adjust the average power of the lamp 45 according to the flow of gas.

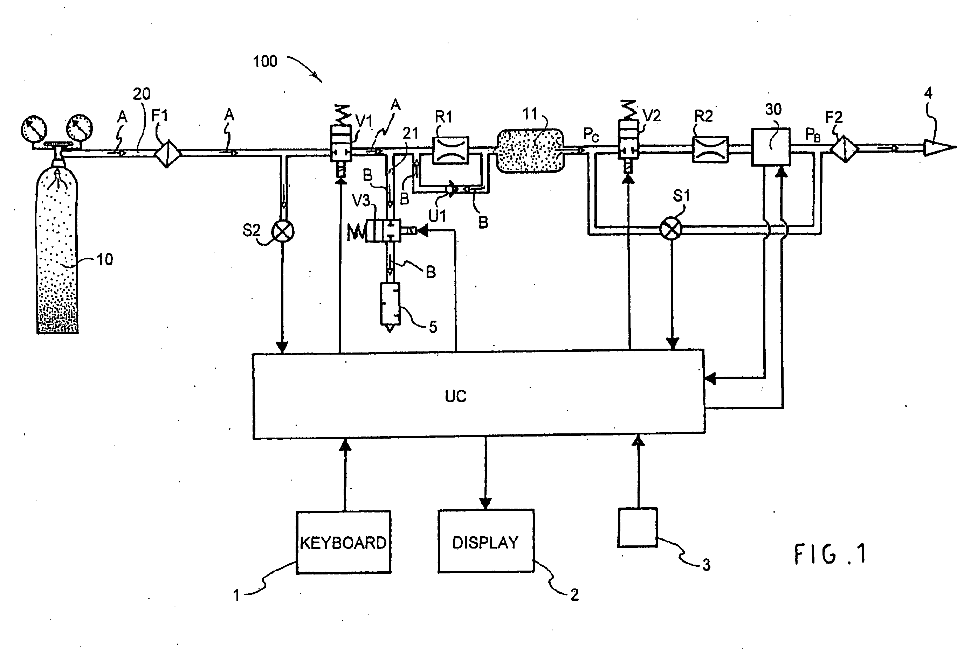

In order to adjust the temperature of the gas leaving the machine 100 in a precise manner that can be controlled by the user, the heating system 30 is realized in two stages. In this manner, by means of the keyboard 1 (Fig. 1), the user can enter a gas temperature value (generally between 20°C and 40°C), which must correspond to the temperature of the gas leaving the machine, when it is injected into the patient's skin. Therefore, if the fist stage is able to provide a thermal jump of about 120°C, it is sufficient for the second stage to provide a limited thermal jump, for example in the range of 5°C to 25°C.

As shown in Figure 2, the first stage comprises the main heater/sterilizer 40 and the first temperature sensor T1 each connected to the control unit UC. The temperature sensor T1 measures the temperature at the outlet of the main heater/sterilizer 40. According to the temperature measured by the sensor T1 the control unit UC controls the electrical power of the lamp 45, so as to have an approximately constant temperature in the range of 10°C to 15°C at the outlet of the main heater/sterilizer 40.

The second stage comprises the post-heater 60 and the second temperature sensor T2, each connected to the control unit UC. The post-heater 60 is of the type with an electrical resistance 61 to allow fine adjustment of the outlet temperature of the gas. The electrical resistance 61 of the post-heater 60 is set so as to allow a limited thermal jump in the range of 5°C to 25°C.

The temperature sensor T2 measures the temperature at the outlet of the post-heater 60. The control unit UC compares the temperature value measured by the sensor T2 with the temperature value set by the user and powers the electrical resistance 61 of the post-heater 60, so as to make the two temperature values coincide.

In this manner, a flow gas at a constant temperature in the range of 20°C to 40°C can be guaranteed at the outlet of the apparatus 100.

As an alternative or in addition to control on the electrical power of the germicidal UV lamp 45 and of the electrical resistance 61, control on the flow of gas inside the heating and sterilization system 30 can be provided. For example a flow-regulating valve can be provided at the outlet of the main heater sterilizer 40 and/or at the outlet of the post-heater 60, so as to regulate the flow of gas within the main heater/sterilizer 40 and/or within the post-heater 60.

Numerous variations and modifications of detail within the reach of a person skilled in the art can be made to the present embodiments of the invention without thereby departing from the scope of the invention as set forth in the appended claims.

**Claims**

1.  An apparatus (100) for subcutaneous delivery of gas for therapeutic purposes, in particular carbon dioxide ($CO_2$), comprising a tank (10) for delivery of the gas connected, by means of a delivery line (20) to a needle (4) designed to be inserted into the patient's subcutaneous tissue, a heating/sterilization system (30) disposed in said delivery line (20), upstream of said injection needle (4), to sterilize said gas and heat it to an optimal temperature to enable it to be injected beneath a patient's skin, **characterized in that** said heating/sterilization system (30) comprises a heater/sterilizer (40) comprising a container (41) containing a UV lamp (45) which emits electromagnetic waves at ultraviolet frequencies and there being defined within said container (41) a chamber (46) through which said gas flows, said chamber (46) of the container (41) being made like a blackbody cavity to convert the high-frequency electromagnetic waves emitted by the UV lamp (45) from the ultraviolet range (between $250\eta m$ and $280\eta m$) to the infrared range (between $5\mu m$ and $20\eta m$).

2.  An apparatus (100) according to claim **1**, **characterized in that** the wall of said chamber (46) of the container (41) is maintained at a temperature of about 30°C, so that within said chamber (46) an infrared emission peak around $10\mu m$ is found.

3.  An apparatus (100) according to claim **1** or **2**, **characterized in that** said container (41) is made of aluminium and its inside surface is treated with a deep oxidation process.

4.  An apparatus (100) according to any one of claims **1** to **3**, **characterized in that** said container (41) is substantially cylindrical and a transparent cylindrical shield (42) is disposed therein so as to define a gas-tight seat (44) inside which said UV lamp (45) is disposed axially and said chamber (46) having a toroidal shape, defined between said transparent shield (42) and said container (41).

5.  An apparatus (100) according to any one of claims **1** to **4**, **characterized in that** said heating/sterilizing system (30) further comprises a temperature control system comprising a temperature sensor (T1) disposed at the outlet of said heater/sterilizer (40) and connected to a control unit (UC) which controls the power of said UV lamp (45) according to the temperature values measured by said sensor (T1).

6.  An apparatus (100) according to any one of claims **1** to **5**, **characterized in that** said heating/sterilizing system (30) further comprises a temperature control system comprising a post-heater (60) with an electrical resistance (61) and a temperature sensor (T2) disposed at the outlet of said post-heater (60) and connected to a control unit (UC) which controls the power of said resistance (61) of the post-heater according to the values measured by said sensor (T2).

7.  An apparatus (100) according to any one of the preceding claims, **characterized in that** it further comprises,

    - flow control means (V1, V2, V3) disposed in said delivery line (20) to control the gas flow, so as to have a constant gas pressure at the outlet of the apparatus,
    - pressure sensor means (S1, S2) to measure the pressure of the gas in said delivery line (20),
    - a user interface (1, 2, 3) to allow the operator to control operation of the apparatus (100), and
    - a control unit (UC) connected to said flow control means (V1, V2, V3), to said pressure sensor means (S1, S2) and to said user interface (1, 2, 3) to control said flow control means (V1, V2, V3) according to the data received from said pressure sensor means (S1, S2) and from said user interface (1, 2, 3).

**Patentansprüche**

1. Vorrichtung (100) zur subkutanen Abgabe von Gas, insbesondere Kohlendioxid ($CO_2$) für therapeutische Zwecke, die einen Tank (10) zur Abgabe des Gases, der, mit Hilfe einer Zufuhrleitung (20) mit einer Nadel (4) verbunden, die dafür gestaltet ist, in das subkutane Gewebe des Patienten eingesetzt zu werden, eine Erhitzungs-/Sterilisationsanlage (30), die in der Zufuhrleitung (20) stromaufwärts von der Injektionsnadel (4) angeordnet ist, um das Gas zu sterilisieren und auf eine optimale Temperatur zu erhitzen, um zu ermöglichen, dass es unter die Haut eines Patienten injiziert wird, **dadurch gekennzeichnet, dass** die Erhitzungs-/Sterilisationsanlage (30) eine Erhitzungs-/Sterilisationseinrichtung (40) umfasst, die einen Behälter (41) umfasst, der eine UV-Lampe (45) enthält, die elektromagnetische Wellen bei Ultraviolettfrequenzen aussendet, und wobei innerhalb des Behälters (41) eine Kammer (46) definiert wird, durch die das Gas strömt, wobei die Kammer (46) des Behälters (41) wie ein Schwarzkörper-Hohlraum hergestellt ist, um die durch die UV-Lampe (45) ausgesendeten hochfrequenten elektromagnetischen Wellen vom Ultraviolettbereich (zwischen 250 nm und 280 nm) zum Infrarotbereich (zwischen 5 $\mu$m und 20 $\mu$m) umzuwandeln.

2. Vorrichtung (100) nach Anspruch 1, **dadurch gekennzeichnet, dass** die Wand der Kammer (46) des Behälters (41) bei einer Temperatur von etwa 30°C gehalten wird, so dass innerhalb der Kammer (46) eine Infrarot-Emissionsspitze um 10 $\mu$m zu finden ist.

3. Vorrichtung (100) nach Anspruch 1 oder 2, **dadurch gekennzeichnet, dass** der Behälter (41) aus Aluminium hergestellt ist und seine Innenfläche mit einem Tiefoxidationsverfahren behandelt ist.

4. Vorrichtung (100) nach einem der Ansprüche 1 bis 3, **dadurch gekennzeichnet, dass** der Behälter (41) im Wesentlichen zylindrisch ist und eine transparente zylindrische Abschirmung (42) in demselben angeordnet ist, um so einen gasdichten Sitz (44) zu definieren, innerhalb dessen die UV-Lampe (45) in Axialrichtung angeordnet ist, und die Kammer (46) eine kreisringförmige Gestalt hat, die zwischen der transparenten Abschirmung (42) und dem Behälter (41) definiert wird.

5. Vorrichtung (100) nach einem der Ansprüche 1 bis 4, **dadurch gekennzeichnet, dass** die Erhitzungs-/Sterilisationsanlage (30) ferner eine Temperaturregelungsanlage umfasst, die einen Temperatursensor (T1) umfasst, der am Auslass der Erhitzungs-/Sterilisationseinrichtung (40) angeordnet und mit einem Steuergerät (UC) verbunden ist, das die Leistung der UV-Lampe (45) entsprechend den durch den Sensor (T1) gemessenen Temperaturwerten regelt.

6. Vorrichtung (100) nach einem der Ansprüche 1 bis 5, **dadurch gekennzeichnet, dass** die Erhitzungs-/Sterilisationsanlage (30) ferner eine Temperaturregelungsanlage umfasst, die eine Nacherhitzungseinrichtung (60) mit einem elektrischen Widerstand (61) und einen Temperatursensor (T2) umfasst, der am Auslass der Nacherhitzungseinrichtung (60) angeordnet und mit einem Steuergerät (UC) verbunden ist, das die Leistung des Widerstands (61) der Nacherhitzungseinrichtung entsprechend den durch den Sensor (T2) gemessenen Werten regelt.

7. Vorrichtung (100) nach einem der vorhergehenden Ansprüche, **dadurch gekennzeichnet, dass** sie ferner Folgendes umfasst:

   - Durchflussregelungsmittel (V1, V2, V3), die in der Zufuhrleitung (20) angeordnet sind, um den Gasdurchfluss zu regeln, um so am Auslass der Vorrichtung einen gleichbleibenden Gasdruck zu haben,
   - Drucksensormittel (S1, S2) zum Messen des Drucks des Gases in der Zufuhrleitung (20),
   - eine Benutzerschnittstelle (1, 2, 3), um zu ermöglichen, dass der Bediener den Betrieb der Vorrichtung (100) steuert, und
   - ein Steuergerät (UC), das mit den Drucksensormitteln (S1, S2), mit den Durchflussregelungsmitteln (V1, V2, V3) und mit der Benutzerschnittstelle (1, 2, 3) verbunden ist, um die Durchflussregelungsmittel (V1, V2, V3) entsprechend den von den Drucksensormitteln (S1, S2) und von der Benutzerschnittstelle (1, 2, 3) empfangenen Daten zu steuern.

**Revendications**

1. Appareil (100) pour l'administration sous-cutanée de gaz à des fins thérapeutiques, en particulier de dioxyde de carbone ($CO_2$), comprenant un réservoir (10) pour distribuer le gaz relié au moyen d'une ligne de distribution (20) vers une aiguille (4) conçue pour être insérée dans le tissu sous-cutané du patient, un système de chauffage/stérilisation (30) disposé dans ladite ligne de distribution (20) en amont de ladite aiguille d'injection (4), pour stériliser ledit gaz et le chauffer à une température optimale pour lui permettre d'être injecté sous la peau d'un patient, **caractérisé en ce que** ledit système de chauffage/stérilisation (30) comprend un chauffage/stérilisateur (40) comprenant un récipient (41) contenant une lampe UV (45) qui émet des ondes électromagnétiques à des fréquences d'ultraviolets et étant définie à l'intérieur dudit récipient (41), une chambre (46) à travers laquelle le gaz s'écoule, ladite chambre (46) du récipient (41) étant réalisée comme une cavité à corps noir pour convertir les ondes électromagnétiques haute fréquence émises par la lampe UV (45) de la gamme d'ultraviolets (entre 250$\eta$m et 280$\eta$m) à la gamme d'infrarouges (entre 5$\mu$m et 20$\mu$m).

2.  Appareil (100) selon la revendication 1, **caractérisé en ce que** la paroi de ladite chambre (46) du récipient (41) est maintenue à une température d'environ 30°C de façon à ce qu'à l'intérieur de ladite chambre (46), un pic d'émission infrarouge d'environ 10$\mu$m ait lieu.

3.  Appareil (100) selon la revendication 1 ou 2, **caractérisé en ce que** ledit récipient (41) est en aluminium et sa surface interne est traitée par un procédé d'oxydation profonde.

4.  Appareil (100) selon l'une quelconque des revendications 1 à 3, **caractérisé en ce que** ledit récipient (41) est essentiellement cylindrique et un blindage cylindrique transparent (42) est disposé dans celui-ci de manière à définir un support étanche au gaz (44) à l'intérieur duquel ladite lampe UV (45) est disposée axialement et ladite chambre (46) ayant une forme toroïdale, défini entre ledit blindage transparent (42) et ledit récipient (41).

5.  Appareil (100) selon l'une quelconque des revendications 1 à 4, **caractérisé en ce que** ledit système de chauffage/stérilisation (30) comprend en outre un système de commande de température comprenant un capteur de température (T1) disposé à la sortie dudit chauffage/stérilisateur (40) et relié à une unité de commande (UC) qui commande la puissance de ladite lampe UV (45) en fonction des valeurs de température mesurées par ledit capteur (T1).

6.  Appareil (100) selon l'une quelconque des revendications 1 à 5, **caractérisé en ce que** ledit système de chauffage/stérilisation (30) comprend en outre un système de commande de température comprenant un post-chauffage (60) avec une résistance électrique (61) et un capteur de température (T2) disposé à la sortie dudit post-chauffage (60) et relié à une unité de commande (UC) qui commande la puissance de ladite résistance (61) du post-chauffage en fonction des valeurs de température mesurées par ledit capteur (T2).

7.  Appareil (100) selon l'une quelconque des revendications précédentes, **caractérisé en ce qu'**il comprend en outre

    - des moyens de commande d'écoulement (V1, V2, V3) disposés dans ladite ligne de distribution (20) pour commander l'écoulement de gaz de manière à avoir une pression de gaz constante à la sortie de l'appareil,
    - des moyens de capteur de pression (S1, S2) pour mesurer la pression du gaz dans ladite ligne de distribution (20),
    - une interface utilisateur (1, 2, 3) pour permettre à l'opérateur de commander le fonctionnement de l'appareil (100), et
    - une unité de commande (UC) reliée auxdits moyens de commande d'écoulement (V1, V2, V3), auxdits moyens de capteur de pression (S1, S2) et à ladite interface utilisateur (1, 2, 3) pour commander lesdits moyens de commande d'écoulement (V1, V2, V3) en fonction des données reçues desdits moyens de capteur de pression (S1, S2) et de ladite interface utilisateur (1, 2, 3).

FIG.1

EP 1 979 029 B1

FIG.2

FIG.3

FIG.4

Temperature VS gas flow

**EP 1 979 029 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050113797 A